# EUROPÄISCHE PATENTSCHRIFT

(11) **EP 1 231 258 B1**
(45) Veröffentlichungstag und Bekanntmachung des Hinweises auf die Patenterteilung: **12.03.2008**
(21) Anmeldenummer: 01129498.0
(22) Anmeldetag: 11.12.2001
(51) Int. Cl.: C12M 1/00, C12M 1/36

(54) **Verfahren zur Befeuchtung eines Nutzraumes in einem Brutschrank sowie Begasungs-Brutschrank**
Process for humidifying the useful space in an incubator and a controlled atmosphere incubator
Procédé pour l'humidification de l'espace utile d'un incubateur et incubateur à atmosphère controlée

(30) Priorität: 09.02.2001 DE 10106349
(43) Veröffentlichungstag der Anmeldung: 14.08.2002
(73) Patentinhaber: Thermo Electron LED GmbH, 63505 Langenselbold (DE)
(72) Erfinder: Reinhardt, Heiko, 63456 Hanau (DE); Pieczarek, Waldemar, 63486 Bruchköbel (DE); Stahl, Hermann, 63165 Mühlheim (DE)
(74) Vertreter: Lang, Friedrich

(56) Entgegenhaltungen:
- DE-C1- 3 815 528
- US-A- 5 853 361
- US-A- 6 117 687

## Beschreibung

Die Erfindung betrifft ein Verfahren zur Befeuchtung eines Nutzraumes in einem Begasungs-Brutschrank, wobei Wasser in einer beheizbaren Wanne im Bodenbereich eines den Nutzraum umfassenden Innenbehälters so lange verdampft, bis eine vorgegebene Temperatur erreicht ist, sowie einen Begasungs-Brutschrank.

Es handelt sich hierbei um ein Verfahren nach dem Grundprinzip der passiven Befeuchtung, wonach mit der temperaturgeregelten Beheizung des Innenbehälters gleichzeitig eine Befeuchtung der Innenbehälter-Atmosphäre durch Verdampfung von Wasser aus einer beheizbaren Wanne erfolgt, bis eine vorgegebene Temperatur erreicht ist und die relative Feuchtigkeit in einem Gleichgewichtszustand (dynamisches Gleichgewicht von Kondensation und Verdampfung) verharrt.

Aus der DE 3815528 C1 ist ein Begasungs-Brutschrank zum Kultivieren von menschlichen oder tierischen Zellen oder Geweben bekannt, der ein mittels einer Tür verschließbares Innengehäuse aufweist, das von einem wärmeisolierenden äußeren Gehäuse umgeben ist, wobei eine Befeuchtungseinrichtung im Bodenbereich des Innengehäuses sowie elektrische Heizkörper unterhalb des Bodens des Innengehäuses und im Bereich der Seitenwände angeordnet sind. Die Befeuchtungseinrichtung ist in Form einer ein Wasserbad aufnehmenden Boden-Wanne ausgebildet, wobei die Heizkörper an der Außenseite des Bodens flächig anliegen und im Bereich der Seitenwände und der Rückwand des Innengehäuses so nach oben abgewinkelt sind, dass sie die Bodenebene des Innengehäuses überragen und so den Übergangsbereich zwischen Boden und Seiten - bzw. Rückwand beheizen. Um eine hohe relative Feuchte im Prüfraum zu erreichen, wird somit eine schnelle und gleichmäßige Aufheizung des Wassers gewährleistet, wobei gleichzeitig eine Kondensatbildung an der Innenwand weitgehend vermieden wird.

Weiterhin ist aus der US 6117687 A ein Brutschrank mit geregelter Innenraum-Atmosphäre bekannt, bei dem eine als Innenraum dienende Kammer von einer mit Wasser gefüllten beheizbaren Umhüllung umgeben ist. Eine den Innenraum abschließende Glastür ist dabei mit einer elektrisch leitenden Beschichtung als Beheizung versehen. Weiterhin werden Störungen aus der Umgebung des Brutschrankes beim Betrieb durch die Regelung kompensiert. Es handelt sich hierbei um einen verhältnismäßig aufwändigen Aufbau.

Die Erfindung stellt sich die Aufgabe, mit verhältnismäßig einfachen Mitteln eine Verkürzung der Feuchteerholzeiten in der Atmosphäre des den Nutzraum umfassenden Innenbehälters zu erzielen, sofern eine Öffnung des Innenbehälters erfolgt ist oder die Umgebungsbedingungen sich stark geändert haben.

Die Aufgabe wird verfahrensgemäß dadurch gelöst, dass nach Öffnung einer Tür zum Innenbehälter deren Öffnungszeit U0 bis zum Verschließen ermittelt und in Abhängigkeit von der Öffnungszeit des Innenbehälters ein Zeitraum U1 vorgegeben wird, in dem die Wanne beheizt wird.

Bevorzugte Ausgestaltungen des Verfahrens sind in den Ansprüchen 2 und 3 angegeben.

Als vorteilhaft erweist es sich, dass auf eine Feuchteregelung verzichtet werden kann; weitere Vorteile des Verfahrens sind darin zu sehen, dass der Inkubator in der Lage ist:
1. auf unterschiedliche Umgebungstemperaturen zu reagieren, ohne dass die relative Feuchte einen Wert von 96% überschreitet und als Folge eine Kondensation im Gerät mit entsprechender Kontaminationsgefahr auftreten kann,
2. auf unterschiedliche zeitliche Verläufe von Temperatur und relativer Feuchte bei unterschiedlichen Beladungen so zu reagieren, dass weder ein vorgegebener Temperaturwert noch die relative Feuchte von 96% überschritten wird,
3. auch bei mehrmaligem Türöffnen verbesserte Feuchteerholzeiten zu gewährleisten, während bei rein passiven Befeuchtungssystemen sich die Feuchteerholzeit mit jedem weiteren Türöffnen verschlechtert und es sehr lange dauern kann, bis das Gerat wieder eine ausgeglichene innere Atmosphäre enthält.

In einer bevorzugten Ausführung des Verfahrens weist der Zeitraum U1 einerseits nur eine Heiz-Phase mit der Laufzeit T2 auf, sofern die Öffnungszeit U0 innerhalb eines vorgegebenen Zeitintervalls T1 liegt, andererseits enthalt der Zeitraum U1 zusätzlich eine Nachheiz-Phase mit der Laufzeit T3, sofern die Öffnungszeit U0 das Zeitintervall T1 überschreitet.; dabei wird die Wanne in der Heizphase der Laufzeit T2 mit einer Intensität beheizt, die vom Zeitintervall zwischen den zwei letzten Öffnungen des Innenbehälters abhängig ist.

Die Aufgabe wird vorrichtungsgemäß für einen Begasungs-Brutschrank mit einem Nutzraum in einem mittels Tür verschließbaren temperaturgeregelten Innenbehälter, der von einem wärmeisolierenden äußeren Gehäuse mit einer äußeren Tür umgeben ist und im Bodenbereich des Innenbehälters eine mit wenigstens einem steuerbaren Heizelement versehene Befeuchtungsvorrichtung für die Innenbehälter-Atmosphäre in Form einer ein Wasserbad aufnehmenden Wanne aufweist, dadurch gelöst, dass die zum Schließen des Innenbehälters vorgesehene Türmit einem Türschalter versehen ist, der mit dem Eingang eines Steuergerätes elektrisch verbunden ist, über das die Energiezufuhr für das wenigstens eine Heizelement ansteuerbar ist.

Bevorzugte Ausgestaltungen des Begasungs-Brutschrankes sind in den Ansprüchen 5 bis 10 angegeben.

Vorteilhafterweise ist das wenigstens eine Heizelement in der Umgebung des Bodenbereichs außerhalb des Innenbehälters angeordnet.

In einer bevorzugten Ausgestaltung des erfindungsgemäßen Brutschranks sind außer dem Heizelement bzw. Heizelementen in der Ebene des Bodenbereichs weitere Heizelemente im Bereich der Seitenwände und der Rückwand des Innenbehälters so angeordnet, dass sie die Bodenebene des Innenbehälters überragen, wobei sich sämtliche Heizelemente außerhalb des Innenbehälters befinden.

Darüber hinaus ist es möglich, die den Innenbehälter abschließende Tür als beheizbare Glastür auszubilden, so dass auch unter ungünstigen Umständen ein Beschlagen der Glasscheibe verhindert wird.

Die Heizelemente der beiden Seitenwände der Rückwand sowie ggf. der beheizbaren Glastür werden nachfolgend zwecks Vereinfachung auch als Wandheizung bezeichnet.

In einer bevorzugten Ausführungsform weist das Steuergerät wenigstens ein Zeitglied auf, mit dessen Hilfe die zeitliche Funktion von Öffnen und Schließen des Türschalters ausgewertet werden kann.

Als vorteilhaft erweist sich, dass der erfindungsgemäße Brutschrank bzw. Inkubator in der Lage ist, auf die unterschiedlichen Längen der Türöffnungen zu reagieren, sowie auf die unterschiedlichen Intervallzeiten zwischen den Türöffnungszeiten zu reagieren und die zugeführte Wärmemenge entsprechend zu steuern.

Weiterhin ist vorteilhafterweise der Türschalter einer den Innenbehälter dicht abschließenden inneren Tür zugeordnet.

In einer vorteilhaften Ausgestaltung des Begasungs-Brutschrankes ist das wenigstens eine Heizelement der Befeuchtungsvorrichtung mittels der Steuereinheit so umsteuerbar, dass es auch als Teil des Regelkreises für die Temperatur-Regelung im Innenbehälter eingesetzt werden kann. Die Umschaltbarkeit erweist sich hier als besonders zweckrnäßige Einsparmöglichkeit im Hinblick auf die Anzahl von Heizelementen. Darüber hinaus sind im Bereich bzw. der Umgebung (Rückwand, Seitenwände) des Innenbehälters Heizelemente vorgesehen, die stets nur als Stellglieder des Regelkreises für die Temperatur-Regelung eingesetzt werden. Auch die beheizbare Glastür kann an die Temperatur-Regelung angeschlossen werden.

Vorzugsweise sind sowohl die Funktion des Steuergeräts als auch die Funktion eines Reglers in einem Digital-Rechner programmiert, woraus sich ein kostengünstiger Aufbau ergibt.

Die Temperaturdifferenz zwischen Sollwert- und Istwert-Temperatur ist abhängig von der Geräteausführung 230/120 Volt und Edelstahl-/Kupfer-lnnenbehälter.

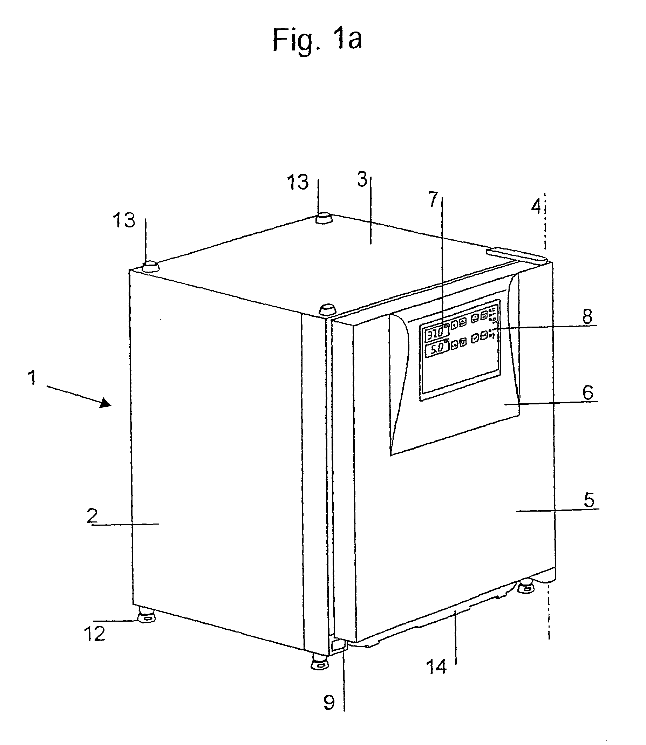

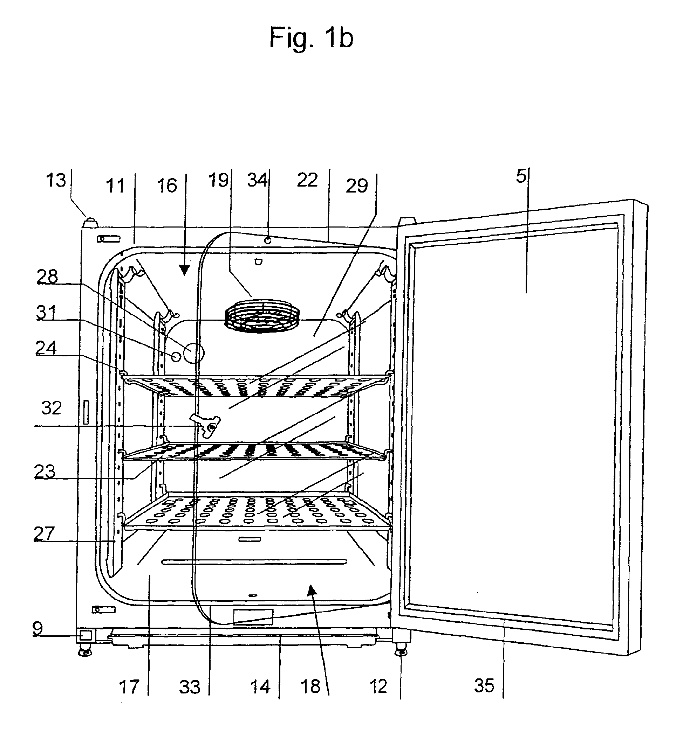

Im folgenden ist der Gegenstand der Erfindung anhand der Figuren 1 a, 1 b, 2 und 3 näher erläutert.
- Figur 1a: zeigt einen geschlossenen Begasungsbrutschrank in räumlicher Darstellung;
- Figur 1b: zeigt in einer perspektivischen Darstellung einen Begasungs-Brutschrank mit einer ein Wasserbad aufnehmenden Boden-Wanne im Innenbehälter, wie es im Wesentlichen bereits aus der DE 3815528 C1 bzw. der entsprechenden EP 0340341 B1 bekannt ist;
- Figur 2: zeigt im Flussdiagramm schematisch die Funktionsweise des erfindungsgemäßen Verfahrens;
- Figur 3: zeigt im Blockschaltbild Funktionseinheiten des erfindungsgemäßen Brutschranks zur Durchführung des Verfahrens, die zum besseren Verständnis als diskrete Bauelemente gezeigt sind, auch wenn sie in der Praxis durch Programme und Datensätze für einen digitalen Rechner ausgeführt sind.

Gemäß Figur 1a weist der Brutschrank ein nach außen abschließbares Gehäuse 1 auf, welches Seitenwände 2, eine hier nicht erkennbare Rückwand sowie Boden- und Deckenplatte 3 aufweist, wobei eine um eine vertikale Achse 4 schwenkbare Fronttür 5 das Gehäuse 1 gegenüber der Umgebungsatmosphäre abschließt. Die Fronttür weist ein Bedienungsfeld 6 mit Anzeigeelementen 7 sowie Betätigungselementen 8 auf, wobei der eigentliche Betriebsschalter 9 im unteren linken Bereich einer die Frontöffnung umfassenden Rahmenplatte 11 angeordnet ist und dieser Bereich von der geschlossenen Fronttür 5 nicht abgedeckt wird. Das Gehäuse 1 des Brutschrankes weist an seiner Unterseite höhenverstellbare Füße 12 auf, welche so ausgebildet sind, dass sie auf Stapelelemente 13 der Deckenplatte eines ggf. bereits vorhandenen Brutschrankgehäuses arretierbar sind. Auf diese Weise ist es möglich, zwei bzw. drei Brutschränke übereinander zu stapeln. Weiterhin ist im unteren Teil des Frontbereiches von Gehäuse 1 ein ausziehbares Ablagefach 14 zur zwischenzeitlichen Aufbewahrung von Proben für den Brutschrank vorgesehen.

Gemäß Figur 1b weist das äußere Gehäuse 1 des Brutschrankes auf seiner Innenseite hier nicht erkennbare Wärmeisolationskörper auf, welche einen Innenbehälter 16 umhüllen, der in seinem Bodenbereich 17 eine ein Wasserbad aufnehmende Wanne 18 enthält. Zur Befeuchtung der Atmosphäre im Innenbehälter 16 wird die Wanne 18 durch im Bodenbereich bzw. im unteren Bereich von Rückwand und Seitenwänden angeordnete Heizelemente erwärmt, so dass in der Atmosphäre des Innenbehälters 16 verhältnismäßig rasch eine hohe relative Feuchte erzeugt werden kann. Die Heizelemente sind vorteilhafterweise in der Umgebung des Bodenbereichs jedoch außerhalb des Innenbehälters 16 angeordnet; dies bedeutet, dass sich die Heizelemente zwischen dem Innenbehälter und dem äußeren Gehäuse 1 befinden, so dass das Innere des Innenbehälters keinerlei Heizelemente aufweist und somit vorteilhafterweise auf einfache Art und Weise gereinigt werden kann.

Zur Kontrolle der Innenraum-Atmosphäre des Innenbehälters 16 ist ein Regelsystem vorgesehen, welches jeweils einen Sensor zur Erfassung der Innenraum-Temperatur und einen CO₂-Sensor zur Erfassung des CO₂-Gehaltes in der Atmosphäre mit jeweils einem eigenen Regelkreis aufweist. Zur Aufnahme der beiden Sensoren ist Messzelle 19 vorgesehen, die sich im Deckenbereich des Innenbehälters 16 befindet, wobei die Messzelle mit einem Lüfterrad versehen ist, so dass die zugeordnete Sensorik (Temperatur- und CO₂-Gehalt Sensor) rasch mit aktuellen Messwerten versorgt werden kann.

Die von den Sensoren ermittelten Messwerte werden in elektrische Signale umgewandelt und als Istwert-Signale mit zuvor eingestellten Sollwert-Signalen verglichen; bei einer Regelabwei chung (Differenz zwischen Sollwert und Istwert) wird ein Stellsignal zur Nachregelung der Tem peratur mittels Wärmezufuhr durch die Heizelemente bzw. des CO₂-Gehaltes mittels Gaszufuhr im Innenraum des Innenbehälters ausgegeben.

Die Regelung des CO₂-Gehaltes im Innenbehälter erfolgt nach Verfahren, wie sie beispielsweise aus der DE-PS 196 57 520, DE-PS 29 24 446 bzw. der DE 33 15 085 C2 bekannt sind.

Der Innenbehälter 16 ist durch eine innere Tür 22 zugänglich, die vorzugsweise zwecks optischer Kontrolle der Kultivierung des im Innenraum befindlichen Guts als (beheizbare) Glastür ausgebildet ist. Weiterhin ist gemäß Figur 1a das äußere Gehäuse 1 mit der äußeren Tür 5 verschließbar, wobei innere und äußere Tür jeweils im Frontbereich des Brutschrankes angeordnet sind.

Weiterhin ist anhand Figur 1b eine beispielhafte Innenausstattung des Innenbehälters 16 mit horizontal angeordneten Einlageblechen 23, die zur Aufnahme von Behandlungs-gut vorgesehen sind, erkennbar. Die Einlagebleche 23 ruhen auf Auflagebügeln 24, die in Öffnungen 26 von vertikal verlaufenden Tragprofilen 27 im Seitenwandbereich arretiert sind.

Im Bereich der Rückwand 29 von Innenbehälter 16 sind eine Rohrdurchführung 28 mit nach innen gerichtetem Gasauslass für die CO₂-Zufuhr ins Innere des Innenbehälters erkennbar.

Weiterhin ist im Bereich von Rückwand 29 eine Druckausgleichsöffnung 31 erkennbar, um für das Ausströmen von verdrängter Luft bei Gaseinlass zu sorgen.

Die als Glastür ausgebildete innere Tür 22 ist mittels eines arretierbaren Handgriffs 32 zu verriegeln, wobei ein anschließendes Verschließen der äußeren Tür nur bei ordnungsgemäßer Verriegelung der inneren Tür möglich ist. Zur Abdichtung des Innenbehälters 16 ist auf der Rahmenplatte 11 eine die Frontöffnung umgebende elastische Dichtung 33 vorgesehen, gegen welche die innere Tür 22 gepresst wird. Weiterhin wird beim Verschließen der inneren Tür 22 ein ebenfalls in der Rahmenplatte 11 befindlicher Türschalter 34 betätigt, so dass mittels Ansteuerung der im Bodenbereich angeordneten Heizelemente eine rasche Wiederherstellung der erwünschten Atmosphäre im Innenbehälter möglich ist.

Anhand Figur 1b ist weiterhin erkennbar, dass die äußere Tür 5 auf ihrer nach innen gerichteten Seite mit einer umlaufenden Dichtung 35 versehen ist, die beim Verschließen der äußeren Tür 5 gegen die Rahmenplatte 11 gepresst wird.

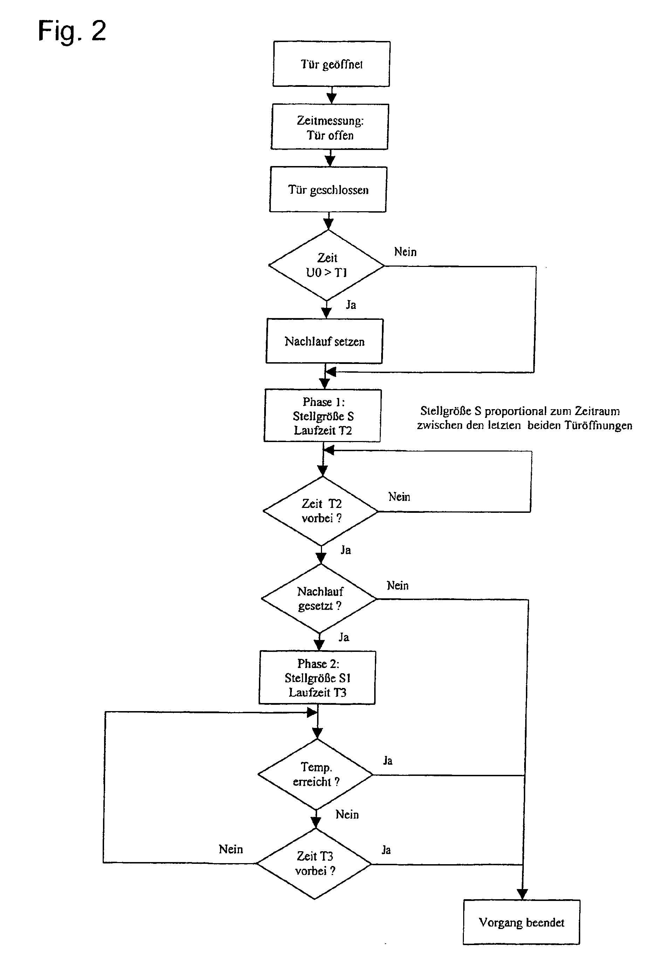

Mit Hilfe von Figur 2 wird nachfolgend eine Störung der Innenraum-Atmosphäre durch Öffnung des Innenbehälters 16 beschrieben.

Bei Öffnung der inneren Tür 22 fällt die Luftfeuchtigkeit im Innenbehälter 16 stark ab, so das die für die Kultivierung des zu behandelnden Guts erforderliche Atmosphäre im Innenraum rasch wieder hergestellt werden muss. Hierzu wird gemäß dem Flussdiagram in Figur 2 zunächst die Öffnungszeit U0 der inneren Tür für den Innenbehälter gemessen und mit einem vorgegebenen Zeitintervall T1 verglichen. Falls die Öffnungszeit U0 innerhalb des vorgegebenen Zeitintervalls T1 liegt, wird die mit Wasser gefüllte Wanne 18 mit der Intensität einer vom Regelkreis unabhängigen Stellgröße S für einen vorgegebenen Zeitraum T2 mittels Heizelement bzw. Heizelementen aufgeheizt, wobei die Stellgröße S vom Zeitraum U3 zwischen den beiden letzten Türöffnungen abhängig ist; in der Praxis wird vorzugsweise eine Stellgröße S verwendet, die dem Zeitraum U3 proportional ist. Falls die Bedingung U0 < T1 gilt, wird am Ende des Zeitraumes T2 die Beheizung mit Stellgröße S beendet und eine Beheizung des Innenraums von Innenbehälter 16 auf der Basis der üblichen Temperaturregelung vorgenommen.

Falls die 0ffnungszeit U0 der Tür 22 für den Innenbehälter 16 das vorgegebene Zeitintervall T1 überschreitet, wird zusätzlich zu Phase T2 eine Nachlaufphase T3 gesetzt, die sich an den Zeitraum T2 anschließt, wobei ebenfalls eine vom Regelkreis unabhängige Stellgröße S1 die Intensität der Beheizung steuert. Die Beheizung mit Stellgröße S1 wird beendet, sobald eine vorgegebene Temperatur im Innenbehälter erreicht ist; die gesteuerte Beheizung mit S1 endet jedoch spätestens mit dem Ablauf der Laufzeitphase T3 unabhängig von der im Innenbehälter 16 herrschenden Temperatur.

Auf die Nachlaufphase T3 kann verzichtet werden, sofern die Regelabweichung (Differenz zwischen Sollwert und Istwert der Temperatur im Innenbehälter) innerhalb eines vorgegebenen Bereichs liegt; in der Praxis liegt dieser Bereich zwischen 0,6°C und 1,4°C je nach Geräteausführung.

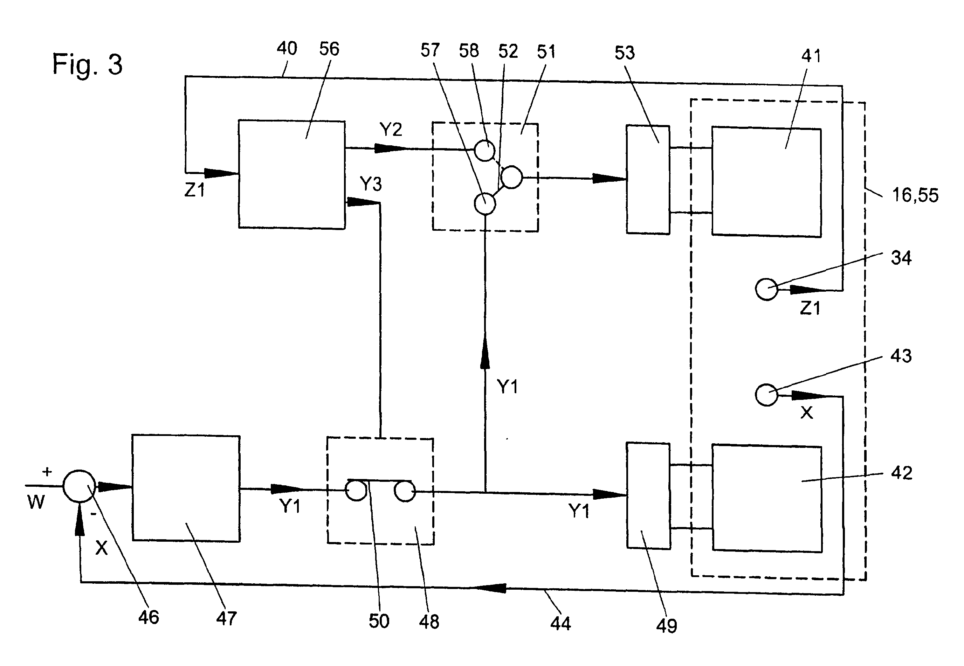

Anhand Figur 3 wird der funktionale Zusammenhang des Verfahrens durch ein Blockschaltbild näher erläutert, wobei die einzelnen Bauelemente bzw. deren Funktionen symbolisch durch Blöcke und deren Verbindungen schematisch dargestellt sind. In der Praxis werden jedoch die Funktionen von Regler, Steuergerät und Umschalter bzw. Unterbrecher von einem programmierbaren Digital-Rechner übernommen, so dass praktisch keine Einzelkomponenten eingesetzt werden.

Nach Figur 3 enthält der Innenbehälter 16 eine Bodenheizung 41, eine Wandheizung 42 (umfasst Heizelemente für Rückwand, linke und rechte Seitenwand und ggf. beheizbare Glastür zum Abschluss des Innenbehälters) sowie einen Temperatursensor 43 (als Teil von Messzelle 19 gemäß Figur 1 b), wobei diese Bauelemente als Teil eines Regelkreises zur Temperaturregelung im Innenbehälter ausgebildet sind. Hierzu wird nach Messung der Temperatur im Innenbehälter durch Temperatursensor 43 ein dem Messwert entsprechendes Istwert-Signal X über Leitung 44 an den invertierenden Eingang eines Reglereingangs 46 geführt, während an dem nicht invertierenden Eingang (Reglereingang 46) der vorher eingestellte Sollwert W anliegt; der zugehörige Regler ist mit Bezugszeichen 47 bezeichnet. Eine eventuelle Regelabweichung des Istwert-Signals X vom vorgegebenen Sollwert W wird dann als Differenzwert (W-X) dem Regler 47 zugeführt, welcher aufgrund seiner Regelcharakteristik (z.B. PID-Regler mit zuvor eingestellten Parametern) ein Stellsignal Y1 ausgibt. Das Stellsignal Y1 wird über einen optionalen Unterbrecherschalter 48 an ein Stellglied 49 weitergeleitet, über welches die Wandheizung 42 betätigt wird; parallel hierzu wird im stationären Regelbetrieb (ohne äußere Störungen) über einen steuerbaren Umschalter 51 mit Kontakt 57 und einen darin befindlichen Schaltkontakt 52 das Stellsignal yl an ein Stellglied 53 für die Ansteuerung der Bodenheizung 41 weitergeleitet. Die Stellglieder 49, 53 sorgen für die Zufuhr elektrischer Energie an die jeweils angeschlossenen Heizungen 42, 41, wobei diese Bauelemente zusammen mit Temperatursensor 43 bei geschlossenem Innenbehälter 16 eine Regelstrecke im Sinne der Regelungstechnik bilden; diese Regelstrecke wird in Figur 3 symbolisch durch den schematisch dargestellten Block 55 umfasst, welcher auch dem Innenraum des schematisch dargestellten Innenbehälters 16 entspricht. Falls nun im stationären Betrieb das aus der Regelstrecke austretende Temperatur-Istwert-Signal X vom Sollwert W abweicht, führt eine solche Regelabweichung (W-X) zu einem am Ausgang des Reglers 47 abgegebenen Stellsignal Y1 entsprechend den Stellgrößen S bzw. S1 gemäß dem Flussdiegramm in Figur 2, wobei das Stellsignal Y1 über die jeweiligen Stellglieder 49 und 53 die Energieversorgung der jeweiligen Heizungen 42 und 41 so lange aktiviert, bis der am Temperatursensor 43 ermittelte Messwert (Istwert X) dem Temperatursollwert W entspricht bzw. die Regelabweichung innerhalb eines vorgegebenen Toleranzbereiches liegt.

Falls nun beispielsweise der Innenbehälter 16 gemäß Figur 1 durch Betätigung der inneren Tür 22 geöffnet wird, wird von dem in Figur 3 symbolisch dargestellten Türschalter 34 ein Störsignal Z1 über Leitung 40 an ein nachgeschaltetes Steuergerät 56 mit eingebautem Zeitglied und Komparator weitergeleitet, wobei das Störsignal Z1 Informationen über Öffnungszeit U0 und Zahl der Öffnungsvorgänge des Innenbehälters 16 gemäß dem Flussdiagramm in Figur 2 enthält.

In den Regelkreis können weitere Störgrößen wie z.B. die Umgebungstemperatur des Brutschranks oder die Anfangstemperatur des zu behandelnden Gutes eingreifen, die mit Z2, Z3 bezeichnet werden, jedoch zwecks besserer Übersicht in Figur 3 nicht gezeigt sind.

Falls nun durch Öffnung der Tür 22 die Feuchtigkeit im Innenbehälter 16 stark abnimmt, wird aufgrund des Störsignals Z1 vom Steuergerät 56 ein Stellsignal Y2 an den Eingang des Umschalters 51 ausgegeben; daraufhin wird der bewegliche Schaltkontakt 52 von der Kontakt-Position 57 für den stationären Regelbetrieb zur Kontakt-Position 58 umgeschaltet, so dass das Stellglied 53 für die Energiezufuhr an die angeschlossene Bodenheizung 41 direkt vom Steuergerät 56 mittels Signal Y2 angesteuert werden kann. Gemäß Figur 2 wird das Stellglied mit einem verhältnismäßig starken Signal S als Stellsignal Y2 angesteuert, woraufhin eine starke Aufheizung der Wasser enthaltenden Wanne 18 (Figur 1b) im Bodenbereich des Innenbehälters 16 zur Erhöhung der Feuchtigkeit erfolgt, wobei gegebenenfalls eine Nachlaufphase mit Stellgröße S1 als Stellsignal Y2 folgt, sofern die Bedingung für das Setzen eines Nachlaufs gemäß Figur 2 erfüllt ist.

Sobald die Nachlaufphasen gemäß Flussdiagramm in Figur 2 beendet sind, wird Umschaltkontakt 52 in seine Ausgangslage auf die Position 57 zurückgeschaltet, so dass nunmehr wieder Stellsignal Y1 aus Regler 47 am Stellglied 53 für eine routinemäßige Ansteuerung der Bodenheizung 41 anliegt.

Weiterhin wird in einer optionalen Ausführungsform von Steuergerät 56 ein Signal aus Türschalter 34 durch Öffnung der inneren Tür bzw. Glastür berücksichtigt, welches als zusätzliches Stellsignal Y3 den zuvor genannten Unterbrecherschalter 48 ansteuert, so dass durch Öffnung eines Umschaltkontaktes 50 das vom Regler 47 ausgehende Stellsignal Y1 unterbrochen wird; d.h., dass die Stellglieder 49 und 53 nicht mehr angesteuert werden können. Dieses optionale Türöffnungssignal ist jedoch nur während der eigentlichen Türöffnungszeit wirksam, so dass nach Schließen der Tür des Innenbehälters wiederum Stellsignal Y1 zumindest an Stellglied 49 anliegt und somit ein Auskondensieren der Feuchtigkeit auf der inneren Wand des Innenbehälters durch Betrieb der Wandheizung 42 vermieden wird. Sobald die Nachlaufphasen gemäß Flussdiagramm Figur 2 abgelaufen sind, kehrt der Regelkreis in seinen ursprünglichen Betriebszustand zurück, wobei nur noch das für den stationären Betrieb charakteristische Stellsignal Y1 den Stellgliedern 49 und 53 für die Wandheizung 42 und die Bodenheizung 41 zugeführt wird.

In einer Ausführungsform des Brutschrankes, bei dem die den Innenbehälter 16 abschließende Tür 22 durch eine Gasblende ersetzt ist, gibt ein auf die äußere Tür ansprechender Türschalter sein Signal an Steuergerät 56, wobei der weitere Ablauf dem Verfahren nach Figur 2 entspricht.

## Patentansprüche

1. Verfahren zur Befeuchtung eines Nutzraumes in einem Begasungs-Brutschrank, wobei Wasser in einer beheizbaren Wanne (18) im Bodenbereich (17) eines den Nutzraum umfassenden Innenbehälters (16) so lange verdampft, bis eine vorgegebene Temperatur erreicht ist,
**dadurch gekennzeichnet,**
**dass** nach Öffnung einer mit einem Türschalter (34) versehenen Tür für den Zugang zum Innenbehälter (16) die Öffnungszeit (U0) der Tür bis zum Verschließen mittels eines Steuergerätes (56), mit dessen Eingang der Türschalter (34) elektrisch verbunden ist, ermittelt wird und in Abhängigkeit von der Öffnungszeit (U0) des Innenbehälters (16) ein Zeitraum (U1) mittels des Steuergeräts (56) vorgegeben wird, in dem mittels des Steuergeräts (56) eine Energiezufuhr für das wenigstens eine Heizelement gesteuert und damit die Wanne beheizt wird.

2. Verfahren nach Anspruch 1,
**dadurch gekennzeichnet,**
**dass** der Zeitraum (U1) nur eine Heizphase mit der Laufzeit (T2) aufweist, sofern die Öffnungszeit (U0) innerhalb eines vorgegebenen Zeitintervalls (T1) liegt und dass der Zeitraum (U1) zusätzlich eine Nachheizphase mit der Laufzeit (T3) enthält, sofern die Öffnungszeit (U0) das Zeitintervall (T1) überschreitet.

3. Verfahren nach Anspruch 2,
**dadurch gekennzeichnet,**
**dass** in der Heizphase der Laufzeit (T2) die Wanne mit einer Intensität beheizt wird, die vom Zeitintervall zwischen den zwei letzten Öffnungen des Nutzraumes abhängig ist.

4. Begasungs-Brutschrank mit einem Nutzraum in einem mittels einer Tür verschließbaren Innenbehälter (16) mit Temperatur-Regelung, wobei der Innenbehälter (16) von einem wärmeisolierenden äußeren Gehäuse (1) mit einer äußeren Tür (5) umgeben ist und im Bodenbereich (17) des Innenbehälters (16) eine mit wenigstens einem steuerbaren Heizelement versehene Befeuchtungsvorrichtung für die Innenbehälter-Atmosphäre in Form einer ein Wasserbad aufnehmenden Wanne (18) vorgesehen ist,
**dadurch gekennzeichnet,**
**dass** die zum Schließen des Innenbehälters (16) vorgesehene Tür (5) mit einem Türschalter (34) versehen ist, der mit dem Eingang eines Steuergeräts (56) elektrisch verbunden ist, über das die Energiezufuhr für das wenigstens eine Heizelement ansteuerbar ist.

5. Begasungs-Brutschrank nach Anspruch 4,
**dadurch gekennzeichnet,**
**dass** das wenigstens eine Heizelement in der Umgebung des Bodenbereichs (17) außerhalb des Innenraums des Innenbehälters (16) angeordnet ist.

6. Begasungs-Brutschrank nach Anspruch 4 oder 5,
**dadurch gekennzeichnet,**
**dass** das Steuergerät (56) wenigstens ein Zeitglied aufweist, mit dessen Hilfe die zeitliche Funktion des Türschalters (34) auswertbar ist.

7. Begasungs-Brutschrank nach einem der Ansprüche 4 bis 6,
**dadurch gekennzeichnet,**
**dass** der Türschalter (34) einer den Innenbehälter (16) dicht abschließenden inneren Tür (22) zugeordnet ist.

8. Begasungs-Brutschrank nach einem der Ansprüche 4 bis 7,
**dadurch gekennzeichnet,**
**dass** die Energiezufuhr für das wenigstens eine Heizelement der Befeuchtungsvorrichtung mittels eines steuerbaren Umschalters (51) umsteuerbar ausgebildet ist, wobei das wenigstens eine Heizelement auch als Teil des Regelkreises für die Temperatur-Regelung im Innenbehälter (16) dient.

9. Begasungs-Brutschrank nach einem der Ansprüche 4 bis 8,
**dadurch gekennzeichnet,**
**dass** in der Umgebung des Innenbehälters (16) Heizelemente vorgesehen sind, die stets als Teil eines Regelkreises für die Temperatur-Regelung des Innenbehälters (16) dienen.

10. Begasungs-Brutschrank nach einem der Ansprüche 4 bis 9,
**dadurch gekennzeichnet,**
**dass** sowohl die Funktion des Steuergerätes (56) als auch die Funktion eines Reglers (47) für die Regelung der Innenraum-Atmosphäre des Innenbehälters (16) in einem Digital-Rechner programmiert sind.

## Claims

1. Method for humidifying a work space in a gas-fed incubator, wherein water in a heatable pan (18) in a floor area (17) of an inner container (16) surrounding the work space is evaporated until a predetermined temperature is reached, wherein after opening of a door with a door switch (34) for access to the inner container (16), an open time (U0) until it is closed is determined by means of a control device (56), whose input is electrically connected to the door switch (34), and a period (U1), during which the pan is heated, is determined depending on the open time (U0) of the inner container (16) by means of the control device (56) that controls a power supply for the at least one controllable heating element so that the pan is heated.

2. Method according to Claim 1, wherein the period (U1) only has a heating phase with a first running time (T2) if the open time (U0) is within a predetermined time interval (T1) and the period (U1) also contains a secondary heating phase with a second running time (T3) if the open time (U0) exceeds the time interval (T1).

3. Method according to Claim 2, wherein during the heating phase with the first running time (T2), the pan is heated at an intensity that is dependent on the time interval between two last openings of the work space.

4. Gas-fed incubator with a work space in an inner container (16) that can be closed by means of a door with temperature control, wherein the inner container (16) is surrounded by a heat-insulating outer housing (1) with an outer door (5), and in a floor area (17) of the inner container (16) there is a humidifier with at least one controllable heating element for an atmosphere of the inner container in the form of a pan (18) holding a water bath, wherein the door (5) for closing the inner container (16) has a door switch (34) that is electrically connected to an input of a control device (56) that can control a power supply for the at least one controllable heating element.

5. Gas-fed incubator according to Claim 4, wherein the at least one heating element is arranged in a region of the floor area (17) outside of an interior of the inner container (16).

6. Gas-fed incubator according to Claim 4 or 5, wherein the control device (56) has at least one time element adapted to evaluate the time function of the door switch (34).

7. Gas-fed incubator according to one of the claims 4 to 6, wherein the door switch (34) is associated with the inner door (22) that tightly seals the inner container (16).

8. Gas-fed incubator according to one of the claims 4 to 7, wherein the power supply for the at least one heating element of the humidifier can be controlled by means of the control device (51) such that the at least one heating element can also be used for temperature control in the inner container (16).

9. Gas-fed incubator according to one of the claims 4 to 8, also comprising heating elements in a region of the inner container (16) used for temperature control in the inner container (16).

10. Gas-fed incubator according to one of the claims 4 to 9, comprising a digital computer wherein a function of the control device (56) and a function of a regulator (47) for control of an interior atmosphere of the inner container (16) are programmed.

## Revendications

1. Procédé pour humidifier un espace utile d'un incubateur à circulation de gaz, dans lequel de l'eau s'évapore dans un bac chauffant (18) dans la zone de fond (17) d'un conteneur intérieur (16) renfermant l'espace utile jusqu'à ce qu'une température prédéterminée soit atteinte, **caractérisé en ce qu'**après l'ouverture d'une porte munie d'un interrupteur de porte (34) pour accéder au conteneur intérieur (16), le temps d'ouverture (U0) de la porte jusqu'à la fermeture est déterminé au moyen d'une unité de commande (56), dont l'entrée communique électriquement à l'interrupteur de porte (34), et en fonction du temps d'ouverture (U0) du conteneur intérieur (16), une période (U1) est déterminée au moyen de l'unité de commande (56), pendant laquelle l'unité de commande (56) commande une alimentation en énergie de l'au mois un élément chauffant et le bac est ainsi chauffé.

2. Procédé selon la revendication 1, **caractérisé en ce que** la période (U1) ne comprend qu'une phase de chauffage ayant la durée (T2) tant que le temps d'ouverture (U0) se situe dans un intervalle de temps (T1) prédéterminé et **en ce que** la période (U1) comprend en outre une phase de post-chauffage ayant la durée (T3) si le temps d'ouverture (U0) dépasse l'intervalle de temps (T1).

3. Procédé selon la revendication 2, **caractérisé en ce que** pendant la phase de chauffage ayant la durée (T2), le bac est chauffé avec une intensité qui dépend de l'intervalle de temps entre les deux dernières ouvertures de l'espace utile.

4. Incubateur à circulation de gaz avec un espace utile dans un conteneur intérieur (16) à température régulée pouvant être fermé au moyen d'une porte, dans lequel le conteneur intérieur (16) est entouré d'un boîtier extérieur (1) isolant thermique avec une porte extérieure (5) et il est prévu dans la zone de fond (17) du conteneur intérieur (16) un dispositif d'humidification de l'atmosphère du conteneur intérieur, muni d'au moins un élément chauffant, sous la forme d'un bac (18) recevant un bain-marie,
**caractérisé en ce que** la porte (5) prévue pour fermer le conteneur intérieur (16) est munie d'un interrupteur de porte (34) qui communique électriquement avec l'entrée d'une unité de commande (56) par laquelle l'alimentation en énergie de l'au moins un élément chauffant peut être activée.

5. Incubateur à circulation de gaz selon la revendication 4, **caractérisé en ce que** l'au moins un élément chauffant est disposé aux environs de la zone de fond (17) à l'extérieur de l'espace intérieur du conteneur intérieur (16).

6. Incubateur à circulation de gaz selon la revendication 4 ou 5, **caractérisé en ce que** l'unité de commande (56) présente au moins un élément temporisateur à l'aide duquel le fonctionnement dans le temps de l'interrupteur de porte (34) peut être évalué.

7. Incubateur à circulation de gaz selon l'une des revendications 4 à 6, **caractérisé en ce que** l'interrupteur de porte (34) est associé à une porte (22) fermant hermétiquement le conteneur intérieur (16).

8. Incubateur à circulation de gaz selon l'une des revendications 4 à 7, **caractérisé en ce que** l'alimentation en énergie de l'au moins un élément chauffant du dispositif d'humidification est conçue pour pouvoir être modifiée par un commutateur commandé (51), l'au moins un élément chauffant servant aussi de partie du circuit de régulation pour la régulation de la température dans le conteneur intérieur (16).

9. Incubateur à circulation de gaz selon l'une des revendications 4 à 8, **caractérisé en ce qu'**il est prévu aux environs du conteneur intérieur (16) des éléments chauffants qui servent aussi de partie d'un circuit de régulation pour la régulation de la température du conteneur intérieur (16).

10. Incubateur à circulation de gaz selon l'une des revendications 4 à 9, **caractérisé en ce que** la fonction de l'unité de commande (56) aussi bien que la fonction d'un régulateur (47) pour la régulation de l'atmosphère de l'espace intérieur du conteneur intérieur (16) sont programmées dans un calculateur numérique.
